# EUROPÄISCHE PATENTSCHRIFT

(11) **EP 3 229 741 B1**
(45) Veröffentlichungstag und Bekanntmachung des Hinweises auf die Patenterteilung: **24.06.2020**
(21) Anmeldenummer: 15807633.1
(22) Anmeldetag: 04.12.2015
(51) Int. Cl.: A61F 2/80, A61F 2/78

(54) **ANPASSBARER PROTHESENSCHAFT FÜR DIE UNTERE EXTREMITÄT**
ADAPTABLE PROSTHESIS SHAFT FOR THE LOWER EXTREMITY
TIGE DE PROTHÈSE ADAPTABLE POUR L'EXTRÉMITÉ INFÉRIEURE

(30) Priorität: 08.12.2014 DE 102014118169; 13.03.2015 DE 102015103689
(43) Veröffentlichungstag der Anmeldung: 18.10.2017
(73) Patentinhaber: Radspieler, Andreas, 83115 Neubeuern (DE)
(72) Erfinder: Radspieler, Andreas, 83115 Neubeuern (DE)
(74) Vertreter: Bobbert & Partner Patentanwälte PartmbB
(86) Internationale Anmeldenummer: PCT/EP2015/078723
(87) Internationale Veröffentlichungsnummer: WO 2016/091763

(56) Entgegenhaltungen:
- WO-A1-2009/012774
- US-A1- 2004 158 332
- US-A1- 2014 277 584

## Beschreibung

Die vorliegende Erfindung betrifft einen Prothesenschaft für die untere Extremität gemäß Anspruch 1.

Beinamputierte Personen können ihre Mobilität mittels Beinprothesen zurückgewinnen. Moderne Beinprothesen umfassen verschiedene Module (Prothesenschaft, Knie-, Unterschenkel-, und Fußmodule), welche kombiniert werden können, um die unterschiedlichsten Bedürfnisse der Prothesenträger (im Folgenden auch kurz als Träger bezeichnet) hinsichtlich Grundmobilität, sportlicher Betätigung und ästhetischen Vorstellungen zu erfüllen. Aus dem Stand der Technik sind unterschiedliche Prothesenschäfte bekannt, wie beispielsweise der modulare Prothesenschaft der US 2014/277584 A1.

Die vorliegende Erfindung betrifft den Prothesenschaft einer Prothese für die unteren Extremitäten, beispielsweise einer Beinprothese. Der Prothesenschaft ist jenes Modul der Prothese, welches die Verbindung zwischen dem mechanischen Ersatz der Extremität und dem verbliebenen Körperteilstumpf (im Folgenden auch kurz als Stumpf bezeichnet) des Prothesenträgers, beispielsweise einem Oberschenkelstumpf, herstellt.

Der Prothesenschaft wird an seinem distalen Ende (jenes Ende, das dem Prothesenträger abgewandt liegt) mit einem mechanischen Ersatz der Extremität, beispielsweise einer modularen Kniegelenk-Unterschenkel-Fuß-Einrichtung für den Oberschenkelamputierten oder einer modularen Unterschenkel-Fuß Einrichtung für den Unterschenkelamputierten, verbunden. An seinem proximalen Ende (jenes Ende, das dem Prothesenträger zugewandt ist) wird der Stumpf in den Prothesenschaft eingeführt. Der Prothesenschaft soll eng und möglichst formschlüssig auf dem Stumpf sitzen. Die passgenaue Verbindung mit dem Körpergliedstumpf bestimmt, wie sicher die Prothese am Stumpf hält.

Zum Übertragen der teils beachtlichen Kräfte zwischen Körper oder Stumpf des Trägers einerseits und der Prothese andererseits, die beim Stehen, Gehen, Aufstehen, Laufen usw. auftreten, bedarf es einer hohen Festigkeit oder Steifheit des Prothesenschafts. Diese wird durch einen entsprechend steifen äußeren Abschnitt des Prothesenschafts, dem so genannten Außenschaft, sichergestellt. Da dessen Steifheit allerdings Druck auf den Stumpf bedingt, was regelmäßig als unangenehm oder gar schmerzhaft empfunden wird und zu Druckstellen führen kann, wird der Außenschaft zur Erhöhung des Tragekomforts in seinem Inneren um einen so genannten Innenschaft (auch als Hülse bekannt) ergänzt. Der Innenschaft weist zumeist ein elastisches Material auf. Er kann in Gestalt einer elastischen Hülle (Liner) hieraus bestehen oder hiermit gepolstert sein.

Eine Aufgabe der vorliegenden Erfindung ist es, einen weiteren Prothesenschaft, oder zumindest einen Außenschaft hierfür, für die untere Extremität vorzuschlagen. Der Prothesenschaft kann Teil einer Beinprothese oder einer Oberschenkelprothese sein.

Die erfindungsgemäße Aufgabe kann durch einen Prothesenschaft mit den Merkmalen des Anspruchs 1 gelöst werden.

Erfindungsgemäß wird somit ein Prothesenschaft für die untere Extremität vorgeschlagen, welcher einen Außenschaft aufweist. Der Außenschaft hat ein Inneres, welches dem Aufnehmen eines Innenschafts hierin dient. Der Innenschaft, auch als Hülse bezeichnet und bekannt, hat ebenfalls ein Inneres, welches wiederum der Aufnahme des nackten oder beispielsweise mit Stoff bekleideten Stumpfs der unteren Extremität dient.

Der Außenschaft hat einen, vorzugsweise elastischen, Umfangsabschnitt.

Der erfindungsgemäße Prothesenschaft umfasst ferner wenigstens eine Anpassungseinrichtung oder weist eine solche auf. Die Anpassungseinrichtung ist Teil des Außenschafts oder mit ihm verbunden oder verbindbar.

Die Anpassungseinrichtung ist ihrerseits ausgestaltet und angeordnet, um bei ihrer Betätigung eine Änderung des Außenschafts zu bewirken oder zuzulassen.

Die Änderung kann die Form des Außenschafts, insbesondere dessen Umfangsform, insbesondere die Form des Umfangsabschnitts, oder die Steifheit oder Festigkeit des Prothesenschafts oder des Außenschafts wenigstens im Bereich des Umfangsabschnitts oder entlang des Umfangsabschnitts betreffen.

Der Außenschaft oder sein Umfangsabschnitt sind ausgestaltet, um trotz, oder ungeachtet, der Betätigung oder der Änderung einen unveränderten Umfang beizubehalten.

Erfindungsgemäße Ausführungsformen können eines oder mehrere der im Folgenden genannten Merkmale in beliebiger Kombination aufweisen, sofern eine konkrete Kombination für den Fachmann nicht als offenkundig technisch unmöglich erkennbar ist. Auch die Gegenstände der Unteransprüche geben jeweils erfindungsgemäße Ausführungsformen an.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Der vorstehend genannte Außenschaft sorgt für die Stabilität des Prothesenschafts und für eine effektive Kraftübertragung, insbesondere im Stehen und Gehen.

Üblicherweise wird der erfindungsgemäße Prothesenschaft mit einem zusätzlichen Innenschaft zum Aufnehmen des Körpergliedstumpfes, insbesondere eines Oberschenkelstumpfes, getragen werden, welcher ebenfalls an die Form und das Volumen des Stumpfes angepasst sein kann. Hierzu weist der Innenschaft vorzugsweise eine elastische Wandung auf oder besteht vollständig oder im Wesentlichen hieraus.

In einigen beispielhaften erfindungsgemäßen Ausführungsformen ist der Innenschaft Teil des Prothesenschafts, in anderen nicht.

In manchen beispielhaften erfindungsgemäßen Ausführungsformen sind Innenschaft und Außenschaft miteinander verbunden, beispielsweise mittels Fügeverfahren wie Kleben, Nieten und dergleichen.

In manchen beispielhaften erfindungsgemäßen Ausführungsformen umschließt der Innenschaft im Wesentlichen oder komplett den gesamten Umfang des Beinstumpfes und nicht nur Teile hiervon.

In einigen beispielhaften erfindungsgemäßen Ausführungsformen verläuft der Umfangsabschnitt gerade oder schräg, beispielsweise relativ zu einer Senkrechten auf die Längsachse des Prothesenschafts in Seitenansicht des Prothesenschafts. Meist wird der Umfangsabschnitt mehr oder weniger (oder genau) in einer Ebene senkrecht zur Längsachse des Prothesenschafts verlaufen.

In manchen beispielhaften erfindungsgemäßen Ausführungsformen befindet sich der Umfang vollständig oder im Wesentlichen in einer Schnittebene.

In weiteren beispielhaften erfindungsgemäßen Ausführungsformen ist der Umfang geschlossen, bzw. bildet eine ununterbrochene Linie oder Struktur.

Der Außenschaft oder sein Umfangsabschnitt sind ausgestaltet, um trotz oder ungeachtet der Änderung, Betätigung oder Anpassung einen unverändertem Umfang beizubehalten. Der unveränderte Umfang bezieht sich in einigen beispielhaften erfindungsgemäßen Ausführungsformen auf einen Umfang einer Öffnung oder einer Öffnungsebene des Außenschafts, durch welche der Innenschaft in den Außenschaft eingeführt wird, hierin auch als Eintrittsöffnung bezeichnet.

Die Anpassungseinrichtung ist Teil des Außenschafts oder mit ihm verbunden oder verbindbar. In einigen beispielhaften erfindungsgemäßen Ausführungsformen wird sie verbunden, indem die Anpassungseinrichtung auf den Außenschaft, und insbesondere auf den Umfangsabschnitt, aufgeschoben wird.

In einigen beispielhaften erfindungsgemäßen Ausführungsformen ist das Betätigen der Anpassungseinrichtung ihr Verschieben in Längsrichtung des Prothesenschafts.

In weiteren beispielhaften erfindungsgemäßen Ausführungsformen weist der Prothesenschaft nur eine Anpassungseinrichtung auf, welche nahe der Öffnungsebene, im Bereich der Öffnungsebene und/oder proximal am Prothesenschaft angeordnet ist.

In einigen beispielhaften erfindungsgemäßen Ausführungsformen weist der Prothesenschaft nur eine Anpassungseinrichtung auf, welche nahe dem Gelenkabschnitt des Prothesenschafts und/oder distal am Prothesenschaft angeordnet ist.

In manchen beispielhaften erfindungsgemäßen Ausführungsformen weist der Prothesenschaft nur eine Anpassungseinrichtung auf, welche zwischen der Öffnungsebene und dem Gelenkabschnitt des Prothesenschafts angeordnet ist.

In manchen beispielhaften erfindungsgemäßen Ausführungsformen ist das Betätigen ein (geringfügiges) Klappen oder Schwenken der Anpassungseinrichtung um eine Achse unter Verändern eines Winkels zwischen einer Längserstreckung der Anpassungseinrichtung und der Längsrichtung des Prothesenschafts.

In einigen beispielhaften erfindungsgemäßen Ausführungsformen werden die Anpassungseinrichtung und/oder der Umfangsabschnitt des Außenschafts mittels Betätigen der Anpassungseinrichtung zwischen wenigstens einer ersten und einer zweiten Position, Stellung oder Zustand veränderbar oder von einer ersten Stellung, Position oder Zustand in eine(n) zweite(n) übergeführt. Das Betätigen kann also ein Überführen sein.

In einigen beispielhaften erfindungsgemäßen Ausführungsformen ist der Zustand eine Festigkeit, insbesondere Biegefestigkeit, eine Elastizität oder eine andere mechanische Größe.

In einigen beispielhaften erfindungsgemäßen Ausführungsformen ist die Anpassungseinrichtung eine Versteifungseinrichtung.

In einigen beispielhaften erfindungsgemäßen Ausführungsformen ist die Anpassungseinrichtung kein Stoffband, welches in seinem Durchmesser veränderbar ist, wie beispielsweise ein Klettband. Eine solche Ausgestaltung einer Anpassungseinrichtung erschwerte beispielsweise das einfache Anpassen des Prothesenschaftes, weil der Träger in aller Regel die Hose ausziehen müsste, um Zugang zu der Anpassungseinrichtung zu haben.

In weiteren beispielhaften erfindungsgemäßen Ausführungsformen ist die Anpassungseinrichtung keine Schraube, kein Klemmhebel, kein Skischuhverschluss und/oder weist vorgenannte Elemente nicht auf.

In manchen beispielhaften erfindungsgemäßen Ausführungsformen ist die Betätigung oder die Änderung dergestalt, dass sie reversibel, umkehrbar und regelmäßig wiederholbar ist.

In gewissen beispielhaften erfindungsgemäßen Ausführungsformen ist die Betätigung oder die Änderung dergestalt, dass sie ohne Werkzeug und/oder ohne Öffnen einer über dem Prothesenschaft liegenden Hose (oder Kleidungsstück allgemein) erfolgen kann.

In einigen beispielhaften erfindungsgemäßen Ausführungsformen weist der Außenschaft ein in eine Wandung des Außenschafts integriertes Stützskelett auf. Das Stützskelett ist biegesteifer als andere Materialien der Wandung wie beispielsweise vergleichsweise weicher Kunststoff, Leder oder ein anderes Material.

Das Stützskelett weist im Bereich des Umfangsabschnitts in Umfangsrichtung eine Vielzahl, z. B. zwei, drei oder mehr, Teilumfangsabschnitte auf oder besteht hieraus. Sind wenigstens zwei dieser Teilumfangsabschnitte in einem Bereich, in welchem sie zueinander benachbart sind, form- und/oder kraftschlüssig verbunden, so ist der Umfangsabschnitt zumindest in diesem Bereich steifer, als wenn der Form- und/oder Kraftschluss aufgehoben wird und die benachbarten Teile nur mittels des sie gemeinsam einbettenden anderen Materials aneinander hängen. Dieses Auflösen des Form- und/oder Kraftschlusses, was aufgrund des anderen Materials nicht mit einer Umfangsänderung einhergeht, wird durch die Anpassungseinrichtung in einigen erfindungsgemäßen beispielhaften Ausführungsformen betätigt. Das Auflösen erlaubt eine bessere Anpassung des Außenschafts an den Körpergliedstumpf in bestimmten Situationen wie dem Hinsetzen des Trägers.

In einigen beispielhaften erfindungsgemäßen Ausführungsformen wird unter dem Umfangsabschnitt, welcher optional elastisch sein kann, ein Außenumfang oder ein Innenumfang des Außenschafts verstanden. In manchen beispielhaften erfindungsgemäßen Ausführungsformen wird unter dem Umfangsabschnitt ein umlaufender Abschnitt verstanden, dessen Dicke der Wandstärke des Außenschafts entspricht.

In einigen beispielhaften erfindungsgemäßen Ausführungsformen weist die Anpassungseinrichtung wenigstens eine Verriegelungseinrichtung auf. Diese ist ausgestaltet, um die Anpassungseinrichtung, den Teilumfangsabschnitt oder den Umfangsabschnitt in wenigstens der ersten oder der zweiten Position, Stellung oder Zustand zu halten.

In einigen beispielhaften erfindungsgemäßen Ausführungsformen ist die Anpassungsvorrichtung keine Schnur und kein Klettband.

In einigen beispielhaften erfindungsgemäßen Ausführungsformen ist die Anpassungseinrichtung ein Dehnabschnitt, ein Scharnier oder Gelenk oder weist ein solches auf. Das Scharnier oder Gelenk kann insbesondere angeordnet sein, um den Teilumfangsabschnitt zwischen der ersten und der zweiten Position bewegbar zu machen oder um ihn klappbar zu machen.

In einigen beispielhaften erfindungsgemäßen Ausführungsformen ist die Anpassungseinrichtung ein ring-fömiger Körper mit geschlossenem Umfang oder weist einen solchen auf. Der ringförmige Körper ist entlang der Längsachse des Außenschafts zwischen wenigstens einer ersten (vorzugsweise) längs-axialen Position und einer zweiten (vorzugsweise längs-axialen) Position verschiebbar.

In bestimmten beispielhaften erfindungsgemäßen Ausführungsformen liegt der ringförmige Körper in der ersten längs-axialen Position außen (zumindest auch) auf dem Umfangsabschnitt auf, oder in radialer Richtung über dem Umfangsabschnitt, in der zweiten Position hingegen nicht (oder jeweils umgekehrt). In diesen beispielhaften Ausführungsformen erhöht der ringförmige Körper in der ersten Position die Festigkeit des Umfangs des Außenschafts zumindest im Bereich des Umfangsabschnitts und verhindert vorzugsweise das Verformen des Umfangsabschnitts des Außenschafts unter Gebrauchsumständen des Prothesenschafts. In der zweiten Position erhöht der ringförmige Körper die Festigkeit des Umfangs des Außenschafts zumindest im Bereich des Umfangsabschnitts nicht. Vorzugsweise lässt er ein solches Verformen in der zweiten Position zu oder verhindert es vorzugsweise zumindest nicht.

Der ringförmige Körper muss keineswegs kreisrund sein. Er gilt vielmehr bereits dann als ringförmig im Sinne der vorliegenden Erfindung, wenn er um eine innenliegende Durchgangsöffnung einen geschlossenen Umfang aufweist oder bildet.

Der ringförmige Körper kann formbeständig sein. Er kann nicht-elastisch sein. Seine Form kann, ohne ihn zerstören zu müssen, jedenfalls unter Einsatzbedingungen für den erfindungsgemäßen Prothesenschaft formbeständig oder fest sein. Der ringförmige Körper kann aus Karbonfasern bestehen oder solche aufweisen.

Der ringförmige Körper kann eine individualisierte Umfangsform aufweisen. Die Individualisierung kann in der Anpassung seiner Form auf eine Umfangskontur des Außenschafts und insbesondere dessen Umfangsabschnitts bestehen. Er kann basierend auf Ergebnissen einer Vermessung des konkreten späteren Trägers des Prothesenschafts gefertigt worden sein.

In bestimmten beispielhaften erfindungsgemäßen Ausführungsformen ist der ringförmige Körper ein bandförmiger Körper, ähnlich einem (in seiner Querschnittsfläche runden oder nicht runden, jedenfalls aber geschlossenen) Zylinderabschnitt.

In manchen beispielhaften erfindungsgemäßen Ausführungsformen weist der ringförmige Körper entlang seines Umfangs eine konstante Höhe auf. Sein oberer Rand und sein unterer Rand liegen dabei vorzugsweise parallel. In anderen beispielhaften erfindungsgemäßen Ausführungsformen ist dies nicht der Fall. Hier liegt insbesondere der proximale Rand, ggf. alternativ oder ergänzend auch der distale Rand, nicht in einer Ebene und/oder verläuft, etwa in einer Seitenansicht des ringförmigen Körpers beurteilt, nicht gerade. Bei diesen Ausgestaltungen ist es vorteilhaft möglich, mittels des ringförmigen Körpers neben dem hierin schwerpunktmäßig betrachteten und diskutierten Umfangsabschnitt auch proximal (oder ggf. distal) zu diesem liegende Abschnitte des Außenschafts zu verstärken. Auf diese Weise ist es möglich, insbesondere Abschnitte des proximalen Rands des Außenschafts mittels der Anpassungseinrichtung weiter zu verstärken, was zu erhöhter Festigkeit und Stabilität beim Tragen der entsprechenden Prothese beitragen kann. Alternativ ist es möglich, diese Abschnitte insbesondere des proximalen Rands des Außenschafts dünner, elastischer oder schwächer auszugestalten als für die gewünschte Stabilität beispielsweise beim Gehen erforderlich, und die fehlende Stabilität mittels der Anpassungseinrichtung zu substituieren. Der hiermit verbundene Vorteil kann darin bestehen, dass der dünner, elastischer oder schwächer ausgestaltete Rand oder Randabschnitt des Außenschafts stets dann, wenn die Anpassungseinrichtung nicht am Rand oder Randabschnitt anliegt, angenehmer für den Träger ist, etwa beim Sitzen. Die an sich jedoch erforderliche Stabilität kann, beispielsweise unmittelbar vor einem Aufstehen, mittels der Anpassungseinrichtung nicht nur für den Umfangsabschnitt sondern eben auch für den o. g. Rand oder Randabschnitt erzielt werden.

In einigen beispielhaften erfindungsgemäßen Ausführungsformen ist der Umfangsabschnitt ein Randabschnitt des Außenschafts oder schließt diesen ein. Der Randabschnitt kann der proximale Abschluss des Außenschafts sein.

Der ringförmige Körper weist in bestimmten beispielhaften erfindungsgemäßen Ausführungsformen keine Einrichtung zum Verstellen seiner Form, der von ihm umschriebenen Fläche oder seiner Umfangslänge auf. Er weist in diesen Ausführungsformen insbesondere keine Schraubverbindung und keine Klettverbindung auf.

Der Umfangsabschnitt kann eine erste Festigkeit oder Elastizität (beispielsweise als Gesamtfestigkeit oder Gesamtelastizität oder als durchschnittliche Gesamtfestigkeit oder Gesamtelastizität ausgedrückt, beispielsweise in der Richtung der größten Erstreckung des Umfangsabschnitts) aufweisen. Der ringförmige Körper kann eine zweite Festigkeit oder Elastizität aufweisen, welche höher ist als die erste, also als jene des Umfangsabschnitts, bei gleichem Vorgehen beim Messen der Festigkeit oder Elastizität.

In bestimmten beispielhaften erfindungsgemäßen Ausführungsformen weist die Verriegelungseinrichtung wenigstens eine Rasteinrichtung auf oder besteht hieraus.

In bestimmten beispielhaften erfindungsgemäßen Ausführungsformen ist die Verriegelungseinrichtung vorzugsweise an einer oder an zwei Außenseiten (lateral, medial) des Außenschafts angeordnet.

In bestimmten beispielhaften erfindungsgemäßen Ausführungsformen ist die Verriegelungseinrichtung ausgestaltet und angeordnet, um den ringförmigen Körper in der ersten und/oder in der zweiten Position zu halten.

In bestimmten beispielhaften erfindungsgemäßen Ausführungsformen ist die Verriegelungseinrichtung an der Anpassungseinrichtung und/oder am Außenschaft angeordnet.

In bestimmten beispielhaften erfindungsgemäßen Ausführungsformen sind die Anpassungsvorrichtung und/oder die Verriegelungseinrichtung lateral am Außenschaft angeordnet.

In bestimmten beispielhaften erfindungsgemäßen Ausführungsformen sind die Anpassungsvorrichtung und/oder die Verriegelungseinrichtung distal am Außenschaft angeordnet.

In bestimmten beispielhaften erfindungsgemäßen Ausführungsformen sind die Anpassungsvorrichtung und/oder die Verriegelungseinrichtung medial am Außenschaft angeordnet.

In bestimmten beispielhaften erfindungsgemäßen Ausführungsformen sind die Anpassungsvorrichtung und/oder die Verriegelungseinrichtung proximal am Außenschaft angeordnet.

In einigen beispielhaften erfindungsgemäßen Ausführungsformen umgibt der Außenschaft den Innenschaft nur abschnittsweise, d. h. dass in einigen Ausführungsformen der Außenschaft nicht die gesamte Fläche des Innenschafts umgibt, sondern nur Abschnitte hiervon. In diesen Ausführungsformen ist wenigstens ein Umfang des Innenschafts vom Außenschaft umgeben. Der Außenschaft kann hierbei eine Art Exoskelett um den Innenschaft bilden.

In einigen erfindungsgemäßen Ausführungsformen ist die Anpassungseinrichtung eine Schiene mit einem Schieber, welche eingerastet oder eingeschoben werden kann.

In bestimmten beispielhaften erfindungsgemäßen Ausführungsformen ist die Änderung der Form eine Zunahme oder Abnahme des M-L-Abstands (medio-lateraler Abstand). In anderen erfindungsgemäßen Ausführungsformen ist sie eine Abnahme oder Zunahme des A-P-Abstandes (antero-posteriorer Abstand).

In bestimmten beispielhaften erfindungsgemäßen Ausführungsformen ist der Umfangsabschnitt vorzugsweise wenigstens eines von: einstückig, geschlossen umlaufend, lückenlos umlaufend, ohne Dopplung im Sinne einer Faltenbildung, ohne Stufe, ohne Spalt und/oder Schlitz. In manchen beispielhaften erfindungsgemäßen Ausführungsformen trifft dies auch für die übrigen Abschnitte des Außenschafts zu.

In gewissen beispielhaften erfindungsgemäßen Ausführungsformen weist der Außenschaft keine über den Umfang verteilten Schalenkörper auf, welche teilweise ineinandergreifen und/oder sich überlappen können.

In manchen beispielhaften erfindungsgemäßen Ausführungsformen weist der Außenschaft Längsschlitze auf, die sich bis in seine proximale Stirnseite ziehen. Dies kann den Tragekomfort erhöhen. Aus Festigkeitsgesichtspunkten ist dies aufgrund der Verstärkung, die mittels der Anpassungseinrichtung im Bereich des Umfangsabschnitts erzielbar ist, vorteilhaft möglich.

In einigen beispielhaften erfindungsgemäßen Ausführungsformen weist der Prothesenschaft keinerlei Sensoren auf.

Einige oder alle erfindungsgemäßen Ausführungsformen können einen oder mehrere der oben oder im Folgenden genannten Vorteile aufweisen.

Der Körpergliedstumpf unterliegt täglich Volumenschwankungen, sowie teils erheblichen Volumenänderungen in den Monaten nach der Amputation, zunächst aufgrund von postoperativen Ödemen und Narbenbildung und später wegen muskulärer Atrophie.

Hinzu kommt die Formänderung des Stumpfs, wenn sich der zunächst stehende Prothesenträger hinsetzt. Üblicherweise nimmt durch Hinsetzen der medio-laterale Abstand des Stumpfes zu, und der antero-posteriore Abstand nimmt ab. Der Querschnitt des Stumpfs geht von, vereinfacht dargestellt, rund oder längs-oval in quer-oval über. Es ist wünschenswert, dass der Prothesenschaft sowohl im Gehen als auch im Sitzen der jeweils aktuellen Stumpfquerschnittsform entspricht, um sowohl ein korrektes Sitzen der Prothese als auch einen hohen Tragekomfort sowohl im Sitzen als auch im Stehen zu gewährleisten. Zudem soll die Prothese insbesondere im Stehen und im Gehen die erforderliche Stabilität gewährleisten. Es ist offensichtlich, dass ein Prothesen-Außenschaft, welcher zum Erfüllen seiner Aufgabe erhebliche Stabilität mitbringen muss, sich aufgrund seiner Festigkeit nicht ohne Weiteres an eine derartige Querschnittsveränderung anpassen kann.

Die vorliegende Erfindung dient vorteilhaft der raschen Anpassung oder Anpassbarkeit des Prothesenschafts auf eine Formänderung des Stumpfs. Die Anpassung kann bequem durch den Träger erfolgen, indem er mit einem oder zwei Handgriffen, die bei vielen erfindungsgemäßen Ausführungsformen durch den Stoff der Hose hindurch erfolgen können, die Festigkeit des Außenschafts seiner Prothese verändert. Der Träger hat hierzu allein die Anpassungseinrichtung zu betätigen. Letzteres kann bei einer ringförmigen Ausgestaltung der Anpassungseinrichtung darin bestehen, sie beim Hinsetzen weiter nach distal und beim Aufstehen weiter nach proximal zu verschieben. Ist die Anpassungseinrichtung klappbar ausgestaltet, so genügt es, ihre Verriegelung zu öffnen und später erneut zu schließen. Diese Anpassungen können durchaus im angezogenen Zustand des Trägers erfolgen, was beispielsweise bei der Verwendung von Klettlösungen oder Schraublösungen, die ein Werkzeug benötigen, nicht möglich ist.

Ein mittels des erfindungsgemäß unveränderten Umfangs des Umfangsabschnitts erzielbarer Vorteil kann darin liegen, dass der Prothesenschaft nach Verringern seiner Festigkeit oder Steifheit nach entsprechendem Betätigen der Anpassungseinrichtung ein Verringern seines Innenvolumens erfahren kann, etwa beim Hinsetzen des Trägers. Der Erfinder der vorliegenden Erfindung hat feststellen können, dass die Veränderung des medio-lateralen Abstands und des anteroposterioren Abstands des Stumpfes, welche sich beim Hinsetzen wie vorstehend diskutiert verändern, was mit einem Übergang von rund oder längs-oval in quer-oval einhergeht, zu einer Veränderung auch des Stumpfvolumens oder seiner Querschnittsfläche führt. Daher sitzt der Stumpf des sitzenden Prothesenträgers in einer anterior-posterior Richtung nicht so fest, wie dies wünschenswert wäre.

Der erfindungsgemäße Prothesenschaft kann diesem Mangel dadurch abhelfen oder zu seinem Verringern beitragen, dass der Umfangsabschnitt, dessen Festigkeit zum Hinsetzen vom Träger verändert werden kann, beim Hinsetzen nicht nur breiter (in medio-lateraler Richtung) sondern auch dünner (in anterior-posteriorer Richtung) wird. Da somit auch die umschriebene Querschnittsfläche des Umfangsabschnitts beim Hinsetzen kleiner werden darf, sitzt die Prothese daher vorteilhaft auch im Sitzen enger und damit besser.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt einen Außenschaft eines erfindungsgemäßen Prothesenschafts einer ersten Ausführungsform für einen rechten Oberschenkelstumpf in einer leicht perspektivischen Ansicht mit Blick von vorne;
- Fig. 2: zeigt einen erfindungsgemäßen Prothesenschaft für einen linken Oberschenkelstumpf beim stehenden Prothesenträger in einer zweiten Ausführungsform;
- Fig. 3: zeigt den erfindungsgemäßen Prothesenschaft der Fig. 2 beim sitzenden Prothesenträger;
- Fig. 4: zeigt schematisch eine Eintrittsöffnung eines erfindungsgemäßen Prothesenschafts für einen rechten Oberschenkelstumpf in leichter Perspektive in einer dritten Ausführungsform;
- Fig. 5: zeigt eine schematische Ansicht einer vierten Ausführungsform des erfindungsgemäßen Prothesenschafts für einen linken Oberschenkelstumpf beim stehenden Prothesenträger; und
- Fig. 6: zeigt in einer Seitenansicht Darstellung des erfindungsgemäßen Prothesenschaft der Fig. 5 beim sitzenden Prothesenträger.

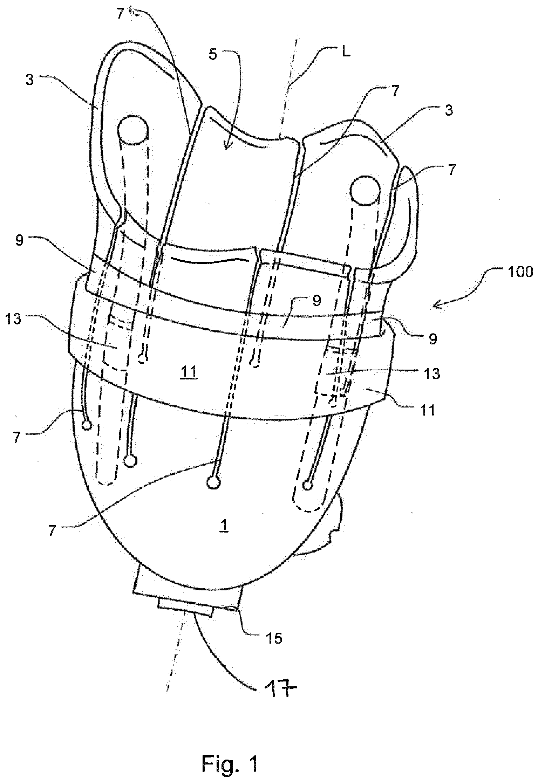

**Fig. 1** zeigt einen Außenschaft 1 eines erfindungsgemäßen Prothesenschafts 100 einer ersten Ausführungsform für einen rechten Oberschenkelstumpf in einer leicht perspektivischen Ansicht mit Blick im Wesentlichen von vorne. Das proximale Ende des Außenschafts 1 liegt in Fig. 1 oben, das distale Ende liegt in Fig. 1 unten. Eine Längsachse des Prothesenschafts 100 oder des Außenschafts ist mit L bezeichnet.

Der Außenschaft 1 bildet eine feste Schale zum passgenauen Aufnehmen des Oberschenkelstumpfs und erlaubt aufgrund seiner Festigkeit und seiner geometrischen Ausgestaltung eine effiziente Kraftübertragung zwischen Oberschenkelstumpf und Prothese. Der Außenschaft 1 ist maßgefertigt und bei sitzendem oder stehendem Träger an die individuelle Form des Oberschenkelstumpfs angepasst.

Der Außenschaft 1 weist einen kelchartigen Korpus auf, welcher proximal mit einem Rand 3 abschließt. Der Rand 3 umschreibt eine Eintrittsöffnung 5 des Außenschafts 1. Durch sie wird der Stumpf, welcher zunächst in einen nicht dargestellten Innenschaft eingeführt sein kann, in den Außenschaft 1 eingeführt.

Ferner weist der Außenschaft 1 bzw. sein Korpus zahlreiche, rein optional vorgesehene, Schlitze 7 auf, welche sich in Längsrichtung des Außenschafts 1 und durch den Rand 3 hinweg erstrecken. Die Schlitze 7 erleichtern ein Verformen des an sich eher festen Außenschafts 1 zum Abbauen oder Verhindern von lokaler Druckbelastung auf den Stumpf.

Dabei bleibt der Umfang des Außenschafts 1 aufgrund des mit ihm im Beispiel der Fig. 1 fest verbundenen Umfangsabschnitts 9 konstant. Der Umfangsabschnitt 9 ist aus einem Material hergestellt, welches zwar seine Umformung oder sein Biegen, jedoch keine Dehnung erlaubt. Hierzu kann er aus Nylonband, aus dem Material für Sicherheitsgurte im Automobilbereich, aus einem Ledergurt oder dergleichen gefertigt sein. Der Umfangsabschnitt 9 ist vorzugsweise nicht elastisch.

Der in seinem Querschnitt verformbare Außenschaft 1 ist für den Prothesenträger erkennbar bequem im Sitzen zu tragen, weil er sich an die quer-ovale Form des Oberschenkelstumpfes anpassen kann. Ohne weitere Vorkehrung wäre der Außenschaft 1 jedoch offensichtlich nicht stabil genug zum Aufnehmen oder Übertragen der in einer stehenden Position des Trägers oder beim Gehen auftretenden Kräfte.

Die vorliegende Erfindung schlägt hierzu das Vorsehen einer Anpassungseinrichtung vor, mit welcher dem Außenschaft 1 beim Übergang beispielsweise zwischen Stehen und Sitzen die hierfür jeweils erforderliche Stabilität vermittelt, der Außenschaft 1 somit also angepasst, werden kann.

Im Beispiel der Fig. 1 weist der Außenschaft 1 zu eben dieser Anpassung eine Anpassungseinrichtung 11 auf.

In der dargestellten Ausführungsform ist die beispielhafte Anpassungseinrichtung 11 ein im Wesentlichen ringförmiger Körper. Dieser umgibt den gesamten Umfang des Außenschafts 1.

Die Anpassungseinrichtung 11 ist aus einem festen, nicht verformbaren Material, beispielsweise aus Karbonfasern, gefertigt. Optimalerweise weist der ringförmige Körper eine Innenkontur auf, welcher der Außenkontur des Außenschafts 1 entspricht. Er ist in solchen Ausführungsformen somit maßgefertigt.

Die Anpassungseinrichtung 11 kann vom Prothesenträger längs der Längsachse L des Außenschafts 1 geschoben werden. Zum Stehen oder Gehen kann der Prothesenträger die Anpassungseinrichtung 11 nach proximal verschieben. Damit erhöht sich die Festigkeit des Außenschaft 1 und insbesondere seines Umfangsabschnitts 9. Verformungen sind in dieser Stellung oder Position im Bereich des Umfangsabschnitts 9 sehr beschränkt oder gar nicht möglich. Zum Hinsetzen kann der Prothesenträger die Anpassungseinrichtung 11 nach distal verschieben. Die Anpassungseinrichtung 11 sitzt dann vergleichsweise locker auf dem Außenschaft 1 und ermöglicht, dass sich dieser bei gleich bleibendem Umfang im Bereich seines Umfangsabschnitts 9 verformt, indem er sich an die Form des Oberschenkelstumpfs im Sitzen anpasst.

Die Anpassungseinrichtung 11 wird mittels einer Verriegelungseinrichtung 13 in der gewünschten Position gehalten. Die Verriegelungseinrichtung 13 kann dafür beispielsweise einen Rastmechanismus aufweisen. Die Verriegelungseinrichtung 13 dient dabei optional als Führungsschiene, welche das Verdrehen und dadurch falsches Positionieren der nicht symmetrischen und deshalb nur ansatzweise "ringförmigen" Anpassungseinrichtung 11 verhindert.

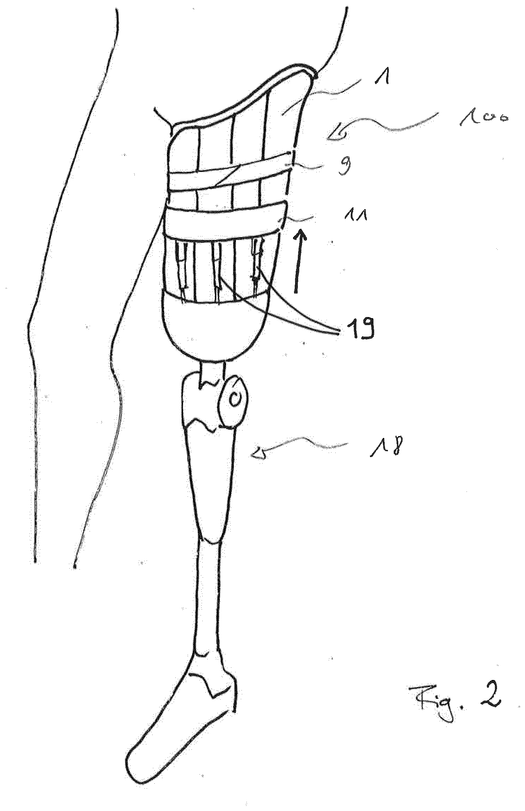

Der Außenschaft 1 umfasst zudem ein Ventil 15 zum Anschließen einer Vakuumpumpe (nicht gezeigt) und ein Verbindungsstück 17 zum Verbinden des Prothesenschafts 100 mit einem künstlichen Kniegelenkmodul. Letzteres ist in Fig. 2 mit dem Bezugszeichen 18 gezeigt.

**Fig. 2** zeigt einen erfindungsgemäßen Prothesenschaft 100 für einen linken Oberschenkelstumpf bei stehendem Prothesenträger in einer zweiten Ausführungsform.

Diese erfindungsgemäße Ausführungsform entspricht im Wesentlichen jener der Fig. 1. Sie unterscheidet sich von jener der Fig. 1, indem die Anpassungseinrichtung 11 entlang mit ihr verbundener Führungsrohre oder -stifte 19 geführt wird.

Die Führungsrohre 19 umfassen wenigstens zwei teleskopartig ineinander gefügte Rohre mit unterschiedlichem Durchmesser, welche konzentrisch angeordnet sein können. Sie werden per Hand oder mittels optional vorgesehenem Motor und/oder Getriebe betätigt. Werden sie auseinander geschoben, so führen sie die Anpassungseinrichtung 11 nach oben in einen Steh-Geh-Zustand des Prothesenschafts 100. Werden sie ineinander geschoben, so nehmen sie die mit ihnen verbundene Anpassungseinrichtung 11 mit nach unten in einen Sitz-Zustand des Prothesenschafts 100.

Bei der manuellen Betätigung dienen die Führungsrohre 19 als passive Führungseinrichtung. Der Prothesenträger schiebt die ringbandförmige Anpassungseinrichtung manuell entlang der Führungsrohre 19 nach unten oder oben.

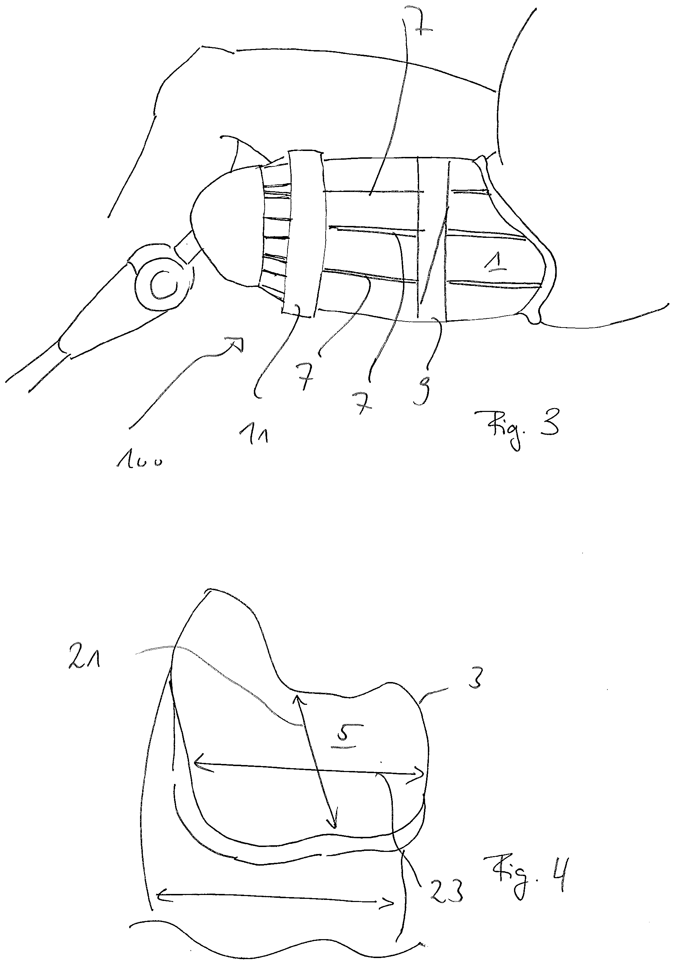

**Fig. 3** zeigt eine laterale Darstellung des erfindungsgemäßen Prothesenschafts 100 der Fig. 2 beim sitzenden Prothesenträger bzw. in der Sitz-Position des Prothesenschafts 100.

Die Anpassungseinrichtung 11 wurde nach distal verschoben, so dass der Außenschaft 1 im Rahmen seiner Grundfestigkeit, welcher nicht zuletzt durch die optionalen Schlitze 7 verformbar ist, sich der Form des Oberschenkelstumpfs im Sitzen vorteilhaft anpassen kann. Dabei bleibt der Umfang des Außenschafts 1 aufgrund des Umfangsabschnittes 9 konstant.

**Fig. 4** zeigt den Blick auf die Eintrittsöffnung 5 eines erfindungsgemäßen Prothesenschafts in einer dritten erfindungsgemäßen Ausführungsform für einen rechten Oberschenkelstumpf in einer leicht perspektivischen Ansicht.

Erkennbar sind der antero-posteriore Abstand 21 und der medio-laterale Abstand 23.

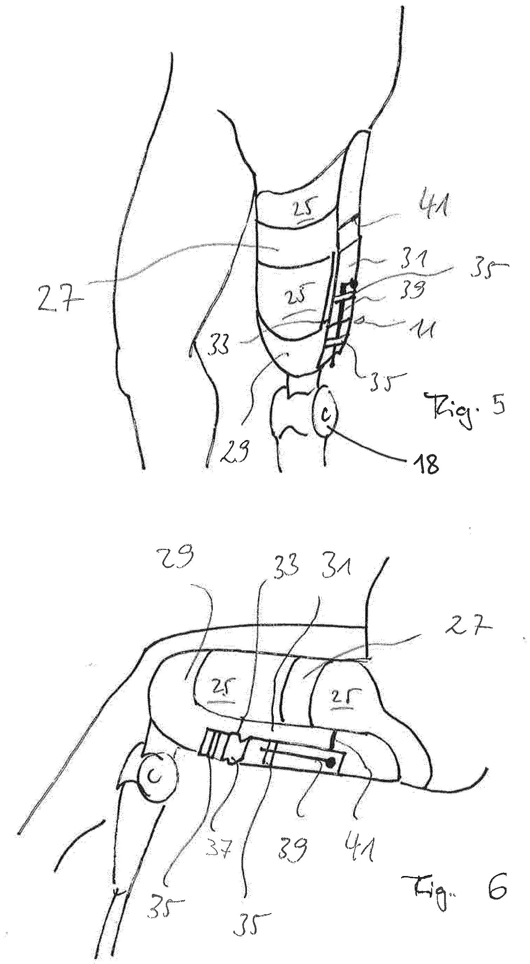

**Fig. 5** und **Fig. 6** zeigen eine schematische Ansicht einer vierten Ausführungsform des erfindungsgemäßen Prothesenschafts 100 für einen linken Oberschenkelstumpf bei stehendem (Fig. 5) bzw. sitzendem (Fig. 6) Prothesenträger.

Der Oberschenkelstumpf ist in einen Innenschaft 25 eingeführt, welcher aus einem elastischen, hautverträglichen Material maßgefertigt ist. Der Innenschaft 25 ist in den Außenschaft 1 eingeführt, welcher in Fig. 5 und 6 als Gerüst oder Stützskelett gezeigt ist, welches aus einem vergleichsweise festen, im Wesentlichen nicht nachgiebigen Material gefertigt ist, welches wiederum in ein undehnbares, aber vergleichsweise verformbares Material eingebettet sein kann. Das vergleichsweise verformbare Material, welches in Fig. 5 und 6 nicht gezeigt ist, ist vorzugsweise nichtdehnbar.

In der dargestellten Ausführungsform weist der Prothesenschaft 100 einen distalen, schalenförmigen Bereich auf, der mit dem Kniegelenkmodul 18 verbunden ist. Der Außenschaft 1 oder sein Gerüst erstreckt sich hiervon ausgehend medial und lateral nach proximal und bildet zwei umlaufende Stützabschnitte 27 und 29.

Beide Stützabschnitte 27, 29 stehen in Kontakt mit einem Längsabschnitt 31 oder sind durch diesen miteinander verbunden.

Der Längsabschnitt 31 des Außenschafts 1 weist wenigstens eine Dehnungslinie 33 auf, welche bei einer offener Anpassungseinrichtung 11, was in Fig. 6 gezeigt ist, ein Verformen des Außenschafts - im Sinne einer Zunahme des M-L-Abstandes und einer entsprechenden Abnahme des A-P-Abstandes - für eine bessere Anpassung des Prothesenschafts 100 an die Form des Oberschenkelstumpfs im Sitzen erlaubt, wie Fig. 6 zeigt.

Statt einer Dehnungslinie 33, oder ergänzend zu dieser, kann ein Schlitz oder eine Unterbrechung des Längsabschnitts 31 vorgesehen sein, welche(r) bei geöffneter Verriegelung (beispielsweise erzielt mittels der Riegelaufnahmen 35 und dem Riegel 39) zu einer geringeren Festigkeit des Außenschafts 1 oder des Längsabschnitts 31 führt, oder hierzu beiträgt, als bei geschlossener Verriegelung.

Die Dehnungslinie 33 und/oder der Schlitz oder die Unterbrechung können alternativ zur Darstellung der Fig. 5 und Fig. 6 auch an anderer Stelle des Längsabschnitts 31 oder des übrigen Außenschafts 1 vorgesehen sein, etwa medial oder weiter proximal als hier für die Dehnungslinie 33 gezeigt.

Die Anpassungseinrichtung 11 weist in der hier gezeigten exemplarischen Ausführungsform wenigstens zwei Riegelführungen oder -aufnahmen 35 auf. Zwischen ihnen befindet sich ein Dehnbereich 37, ein Scharnier oder ein Gelenk. Mittels eines Riegels 39 lässt sich der Dehnbereich 37 verriegeln, wie in Fig. 5 gezeigt, oder eben entriegeln, wie in Fig. 6 gezeigt.

Fig. 5 zeigt somit die Steh-Geh-Position des Prothesenschaftes 100, in welchem der Riegel 39 ein Dehnen im Bereich der Dehnlinie 33 verhindert. In der Fig. 6 ist der Riegel 39 aus wenigstens einer Riegelaufnahme 35 heraus gezogen, so dass er nicht über dem Dehnbereich 37 liegt. Der Außenschaft 1 kann sich nun verformen und dem Oberschenkelstumpf anpassen, da sich die Festigkeit der Stützabschnitte 27, 29 geändert hat. Da sich deren Umfang jedoch nicht ändert, können sie als unveränderliche Umfangsabschnitte 9 gelten.

Die Fig. 5 und Fig. 6 zeigen ferner einen zusätzlichen Gelenkabschnitt 41. Dieser ist in der beispielhaften Ausführungsform der Fig. 5 und 6 proximal zur Dehnungslinie 33 vorgesehen. Er ist lateral am Außenschaft 1 vorgesehen, könnte aber ebenso gut medial, oder lateral und medial, am Außenschaft 1 vorliegen.

Der Gelenkabschnitt 41 kann im Längsabschnitt 31 angeordnet sein. Manch andere Position ist ebenfalls erfindungsgemäß vorgesehen.

Der Gelenkabschnitt 41 kann eine Federeinrichtung aufweisen, beispielsweise eine Spannfeder oder eine Blattfeder. Sie kann angeordnet sein, um die mittels des Gelenkabschnitts 41 miteinander verbundenen Teilabschnitte des Längsabschnitts 31 in einer vorbestimmten Stellung zueinander zu halten. Druck, welcher gegen die Federeinrichtung oder den Gelenkabschnitt 41 wirkt, kann ein Öffnen des Gelenkabschnitts 41 gegen die Federwirkung der Federeinrichtung bewirken. Stammt der Druck vom Stumpf, etwa beim Hinsetzen, so kann ein solches Öffnen als entlastend empfunden werden.

Wichtig ist, dass der Gelenkabschnitt 41 als optionale Ergänzung jener vorstehend diskutierten Anpassungseinrichtung 11 und ihrer optionalen Verriegelungseinrichtung 13, 35, 39 verstanden wird.

Statt eines Gelenkabschnitts 41 können zwei oder mehr derartige Gelenkabschnitte 41 vorgesehen sein.

Es ist offensichtlich, dass der Gelenkabschnitt 41 mit den Merkmalen jeder erfindungsgemäßen Ausführungsform kombiniert werden kann, so z. B. mit jener der Fig. 1 bis 3.

## Patentansprüche

1. Prothesenschaft (100) für die untere Extremität mit einem Außenschaft (1) zum Aufnehmen eines Innenschafts (25) in einem Inneren hiervon, wobei der Innenschaft (25) wiederum zum Aufnehmen eines Körpergliedstumpfs der unteren Extremität in einem Inneren hiervon ausgestaltet ist,
wobei der Außenschaft (1) einen Umfangsabschnitt (9) aufweist,
wobei der Prothesenschaft (100) ferner wenigstens eine Anpassungseinrichtung (11) umfasst oder aufweist, welche Teil des Außenschafts (1) ist oder mit dem Außenschaft (1) verbunden oder verbindbar ist,
**dadurch gekennzeichnet, dass**
die Anpassungseinrichtung (11) ausgestaltet und angeordnet ist, um bei ihrer Betätigung eine Änderung der Form des Außenschafts (1) oder eine Änderung einer Steifigkeit des Prothesenschafts (100) oder des Außenschafts (1) wenigstens im Bereich des Umfangsabschnitts (9) bei unverändertem Umfang des Umfangsabschnitts (9) zu bewirken oder zuzulassen.

2. Prothesenschaft (100) nach Anspruch 1, wobei die Anpassungseinrichtung (11) oder der Umfangsabschnitt (9) des Außenschafts (1) mittels Betätigens der Anpassungseinrichtung (11) zwischen wenigstens einer ersten und einer zweiten Position, Stellung oder Zustand veränderbar oder von einer ersten Stellung, Position oder Zustand in eine(n) zweite(n) überführbar ist.

3. Prothesenschaft (100) nach Anspruch 2, wobei der Außenschaft (1) ein in eine Wandung des Außenschafts (1) integriertes Stützskelett aufweist, welches im Bereich des Umfangsabschnitts (9) in Umfangsrichtung aus einer Vielzahl Teilumfangsabschnitten besteht oder solche aufweist.

4. Prothesenschaft (100) nach einem der Ansprüche 1 oder 2, wobei der Außenschaft (1) ein in eine Wandung des Außenschafts (1) integriertes Stützskelett aufweist, wobei der Außenschaft (1) oder sein Stützskelett wenigstens einen Längsabschnitt (31) mit wenigstens einer Dehnungslinie (33) oder einer Unterbrechung aufweist.

5. Prothesenschaft (100) nach Anspruch 3, wobei der Außenschaft (1) oder sein Stützskelett wenigstens einen Längsabschnitt (31) mit wenigstens einer Dehnungslinie (33) oder einer Unterbrechung aufweist.

6. Prothesenschaft (100) nach einem der Ansprüche 3, 4 oder 5, wobei die Anpassungseinrichtung (11) wenigstens eine Verriegelungseinrichtung (13, 35, 39) aufweist, welche ausgestaltet ist, um die Anpassungseinrichtung (11), den Teilumfangsabschnitt, den Längsabschnitt (31) oder den Umfangsabschnitt (9) in wenigstens der ersten oder der zweiten Position, Stellung oder Zustand zu halten.

7. Prothesenschaft (100) nach einem der vorangegangenen Ansprüche, wobei die Anpassungseinrichtung (11) ein Scharnier oder Gelenk ist oder aufweist.

8. Prothesenschaft (100) nach einem der vorangegangenen Ansprüche, wobei die Anpassungseinrichtung (11) ein ring-fömiger Körper mit geschlossenem Umfang ist oder einen solchen aufweist, wobei der ring-förmige Körper entlang der Längsachse (L) des Außenschafts (1) zwischen wenigstens einer ersten Position und einer zweiten Position verschiebbar ist.

9. Prothesenschaft (100) nach einem der Ansprüche 6 bis 8, wobei die Verriegelungseinrichtung (13, 35, 39) wenigstens eine Rasteinrichtung ist oder aufweist, mittels welcher die Anpassungseinrichtung (11) in der ersten und/oder in der zweiten Position gehalten werden kann.

10. Prothesenschaft (100) nach einem der vorangegangenen Ansprüche, wobei der Außenschaft (1) oder sein Längsabschnitt (31) wenigstens einen Gelenkabschnitt (41) aufweist.

11. Prothesenschaft (100) nach Anspruch 10, wobei der Gelenkabschnitt (41) eine Federeinrichtung aufweist.

12. Prothesenschaft (100) nach einem der vorangegangenen Ansprüche, wobei die Verriegelungseinrichtung (13, 35, 39) an der Anpassungseinrichtung (11) und/oder an dem Außenschaft (1) angeordnet ist.

13. Prothesenschaft (100) nach einer der vorangegangenen Ansprüche, wobei die Anpassungsvorrichtung (11) und/oder die Verriegelungseinrichtung (13, 35, 39) lateral und/oder medial am Außenschaft (1) angeordnet sind.

14. Prothesenschaft (100) nach einer der vorangegangenen Ansprüche, wobei die Anpassungsvorrichtung (11) und/oder die Verriegelungseinrichtung (13, 35, 39) proximal und/oder distal am Außenschaft (1) angeordnet ist.

## Claims

1. A prosthesis shaft (100) for the lower limb having an outer shaft (1) for receiving an inner shaft (25) in an interior thereof, the inner shaft (25), in turn, being designed to receive a limb stump of the lower limb in an interior thereof,
wherein the outer shaft (1) comprises a peripheral portion (9),
wherein the prosthesis shaft (100) further includes or comprises at least one adapter (11), which is part of the outer shaft (1) or is connected or connectable with the outer shaft (1),
**characterized in that**
the adapter (11) is designed and arranged so as to effect or allow, when it is actuated, a modification of the shape of the outer shaft (1) or a modification of a rigidity of the prosthesis shaft (100) or of the outer shaft (1) at least in a region of the peripheral portion (9) with unchanged periphery of the peripheral portion (9).

2. The prosthesis shaft (100) according to claim 1, wherein the adapter (11) or the peripheral portion (9) of the outer shaft (1) is variable between at least a first and a second position, location or state, or is transferable from a first to a second position, location or state by actuating the adapter (11).

3. The prosthesis shaft (100) according to claim 2, wherein the outer shaft (1) comprises a support skeleton integrated into a wall of the outer shaft (1), which consists of or comprises a plurality of partial peripheral portions in a region of the peripheral portion (9) in a peripheral direction.

4. The prosthesis shaft (100) according to anyone of claims 1 or 2, wherein the outer shaft (1) comprises a support skeleton integrated into a wall of the outer shaft (1), wherein the outer shaft (1) or its support skeleton comprises at least one longitudinal portion (31) with at least one strain line (33) or one interruption.

5. The prosthesis shaft (100) according to claim 3, wherein the outer shaft (1) or its support skeleton comprises at least one longitudinal portion (31) with at least one strain line (33) or one interruption.

6. The prosthesis shaft (100) according to anyone of claims 3, 4 or 5, wherein the adapter (11) comprises at least one locking device (13, 35, 39) designed to hold the adapter (11), the partial peripheral portion, the longitudinal portion (31) or the peripheral portion (9) in at least the first or the second position, location or state.

7. The prosthesis shaft (100) according to anyone of the preceding claims, wherein the adapter (11) is or comprises a hinge or a joint.

8. The prosthesis shaft (100) according to anyone of the preceding claims, wherein the adapter (11) is or comprises a ring-shaped body having a closed periphery, the ring-shaped body being slidable along the longitudinal axis (L) of the outer shaft (1) between at least a first position and a second position.

9. The prosthesis shaft (100) according to anyone of claims 6 to 8, wherein the locking device (13, 35, 39) is or comprises at least one snap-in locking device by means of which the adapter (11) can be held in the first and/or in the second position.

10. The prosthesis shaft (100) according to anyone of the preceding claims, wherein the outer shaft (1) or its longitudinal portion (31) comprises at least one joint portion (41).

11. The prosthesis shaft (100) according to claim 10, wherein the joint portion (41) comprises a spring device.

12. The prosthesis shaft (100) according to anyone of the preceding claims, wherein the locking device (13, 35, 39) is arranged on the adapter (11) and/or on the outer shaft (1) .

13. The prosthesis shaft (100) according to anyone of the preceding claims, wherein the adapter (11) and/or the locking device (13, 35, 39) is/are arranged laterally or medially on the outer shaft (1).

14. The prosthesis shaft (100) according to anyone of the preceding claims, wherein the adapter (11) and/or the locking device (13, 35, 39) is/are arranged proximally and/or distally on the outer shaft (1).

## Revendications

1. Une tige de prothèse (100) pour membre inférieur ayant une tige externe (1) pour recevoir une tige interne (25) à l'intérieur de celle-ci, la tige interne (25) étant, à son tour, conçue pour recevoir un moignon de membre inférieur à l'intérieur de celle-ci,
où la tige externe (1) comprend une section périphérique (9),
où la tige de prothèse (100) inclut ou comprend en outre au moins un adaptateur (11) faisant partie de la tige externe (1) ou étant relié ou reliable à la tige externe (1),
caratérisée en ce que
l'adaptateur (11) est conçu et agencé pour effectuer ou permettre, lors de son actionnement, une modification de la forme de la tige externe (1) ou une modification d'une rigidité de la tige de prothèse (100) ou de la tige externe (1) au moins dans une région de la section périphérique (9), la périphérie de la section périphérique (9) restant inchangée.

2. La tige de prothèse (100) selon la première revendication, où l'adaptateur (11) ou la section périphérique (9) de la tige externe (1) est variable entre au moins une première et une seconde position, un premier et un second emplacement ou état, ou est transférable d'une première à une seconde position, d'un premier à un second emplacement ou état, par actionnement de l'adaptateur (11).

3. La tige de prothèse (100) selon la revendication 2, où la tige externe (1) comprend un squelette de support intégré dans une paroi de la tige externe (1), lequel est constitué d'une, ou comprend une pluralité de sections périphériques partielles dans une région de la section périphérique (9) dans la direction périphérique.

4. La tige de prothèse (100) selon l'une quelconque des revendications 1 ou 2, où la tige externe (1) comprend un squelette de support intégré dans une paroi de la tige externe (1), où la tige externe (1) ou son squelette de support comprend au moins une section longitudinale (31) avec au moins une ligne de dilatation (33) ou une interruption.

5. La tige de prothèse (100) selon la revendication 3, où la tige externe (1) ou son squelette de support comprend au moins une section longitudinale (31) avec au moins une ligne de dilatation (33) ou une interruption.

6. La tige de prothèse (100) selon l'une quelconque des revendications 3, 4 ou 5, où l'adaptateur (11) comprend au moins un dispositif de verrouillage (13, 35, 39) conçu pour maintenir l'adaptateur (11), la section périphérique partielle, la section longitudinale (31) ou la section périphérique (9) au moins dans la première ou la seconde position, le premier ou le second emplacement ou état.

7. La tige de prothèse (100) selon l'une quelconque des revendications précédentes, où l'adaptateur (11) est ou comprend une charnière ou une articulation.

8. La tige de prothèse (100) selon l'une quelconque des revendications précédentes, où l'adaptateur (11) est ou comprend un corps annulaire ayant une périphérie fermée, le corps annulaire pouvant coulisser le long de l'axe longitudinal (L) de la tige externe (1) entre au moins une première et une seconde position.

9. La tige de prothèse (100) selon l'une quelconque des revendications 6 à 8, où le dispositif de verrouillage (13, 35, 39) est ou comprend au moins un dispositif d'encliquetage au moyen duquel l'adaptateur (11) peut être maintenu dans la première et/ou dans la seconde position.

10. La tige de prothèse (100) selon l'une quelconque des revendications précédentes, où la tige externe (1) ou sa section longitudinale (31) comprend au moins une section d'articulation (41).

11. La tige de prothèse (100) selon la revendication 10, où la section d'articulation (41) comprend un dispositif à ressort.

12. La tige de prothèse (100) selon l'une quelconque des revendications précédentes, où le dispositif de verrouillage (13, 35, 39) est agencé sur l'adaptateur (11) et/ou sur la tige externe (1).

13. La tige de prothèse (100) selon l'une quelconque des revendications précédentes, où l'adaptateur (11) et/ou le dispositif de verrouillage (13, 35, 39) est/sont agencé(s) de manière latérale ou médiale sur la tige externe (1).

14. La tige de prothèse (100) selon l'une quelconque des revendications précédentes, où l'adaptateur (11) et/ou le dispositif de verrouillage (13, 35, 39) est/sont agencé(s) de manière proximale et/ou distale sur la tige externe (1).
